Europäisches Patentamt

European Patent Office

Office européen des brevets

(11) Publication number: **0 481 459 A1**

# EUROPEAN PATENT APPLICATION

(21) Application number: **91117664.2**

(22) Date of filing: **16.10.91**

(51) Int. Cl.5: **A61M 16/00**

(30) Priority: **19.10.90 IT 2179790**

(43) Date of publication of application:
**22.04.92 Bulletin 92/17**

(84) Designated Contracting States:
**DE FR GB NL SE**

(71) Applicant: **DAR S.p.A.**
**Via Galvani, 6, P.O. Box 78**
**I-41037 Mirandola (Modena)(IT)**

(72) Inventor: **Veronesi, Mario**
**Via Fulvia 21/A**
**I-41037 Mirandola (Province Modena)(IT)**

(74) Representative: **Modiano, Guido et al**
**MODIANO, JOSIF, PISANTY & STAUB**
**Modiano & Associati Via Meravigli, 16**
**I-20123 Milano(IT)**

(54) **Apparatus for continuous positive airway pressure breathing (CPAP).**

(57) The present invention relates to an apparatus for continuous alveolar positive pressure breathing which comprises, downstream of an air and oxygen mixer (1-4), a flow divider (10) which is connected to a variable-volume constant pressure maintenance unit (20) and to a humidifier (13). A merge connection (21) is provided, downstream of the maintenance unit (20) and of the humidifier (13), for connection to an inspirator line (22) of a patient. A pressure control valve (26) is arrangeable on the expirator line (25) of a patient.

Fig.1

The present invention relates to an apparatus for continuous alveolar positive pressure breathing.

As known, some forms of respiratory insufficiency are characterized, initially or during their development, by hypo- or normocapnia associated with severe hypoxia.

The patient is capable of performing respiratory work and respiratory aid must be aimed predominantly at correct hypoxemia.

In these cases, the method of oxygen treatment is capable of solving hypoxemia only if it is associated with a positive pressure which returns the respiratory system to its normal value of functional residual capacity.

This method currently uses an apparatus which substantially comprises an air/oxygen mixer for determining the inspired oxygen fraction, which produces a stream of gas fed onto a heating humidifier.

A reserve and compensation tank is provided on the inspirator line downstream of the humidifier and is normally constituted by an elastic bag or bladder which allows to keep the pressure of the system substantially constant in all the inspirator conditions of the patient.

A Y-shaped connector for the patient is furthermore provided on the inspirator line; a pressure gauge acts on said connector to determine the pressure, and an expirator line, on which a water valve for pressure control is mounted, is connected thereto.

Although this apparatus is in widespread use, it has severe disadvantages, not least of which is the fact that the humidifier is not sized and manufactured for this particular type of use, so there may be moments during which a very high flow of gas occurs and said gas in practice is not adequately treated by the humidifier.

The apparatus is furthermore considerably bulky, also due to the fact that the reserve and compensation tank is generally constituted by an elastic bag with a capacity of approximately thirty liters, which uses its intrinsic elasticity to generate the necessary pressure on the gas; this type of tank, which has been found to be relatively valid for pressures at a certain level, is generally fully insufficient in case of low operating pressures.

Another disadvantage is furthermore constituted by the fact that by using a control valve of the water type, which must be arranged relatively close to the patient, a severe form of contamination is produced, since bacteria and germs are retained by the water which in practice remains free in the environment, consequently creating severe potential dangers.

Another disadvantage is furthermore constituted by the fact that apparatuses of the above described type require constant monitoring of the patient on the part of an operator to check whether the patient begins apnea, since there are no controls capable of automatically indicating this potential danger condition.

The aim of the invention is indeed to solve the above described problems by providing an apparatus for continuous alveolar positive pressure breathing which allows to drastically reduce dimensions while maintaining the possibility of exerting a pressure on the gas which can be set and adjusted according to the contingent requirements.

Within the scope of the above aim, a particular object of the invention is to provide an apparatus wherein the air humidification step is not subject to discontinuities, since the flow of air or gas which is fed to the humidifier is a direct function of the capacity of the humidifier itself, independently of the air absorption factors generated by the patient.

Another object of the present invention is to provide an apparatus which does not produce contamination sources and which furthermore avoids the need for continuous monitoring of the patient on the part of the operator, since it is provided with automatic monitoring systems.

Not least object of the present invention is to provide an apparatus which, by virtue of its particular characteristics, is capable of giving the greatest assurances of reliability and safety in use.

This aim, these objects and others which will become apparent hereinafter are achieved by an apparatus for continuous alveolar positive pressure breathing, characterized in that it comprises, downstream of an air or oxygen mixer, a flow divider connected to a variable-volume constant pressure maintenance unit and to a humidifier, a merge connection being provided, downstream of said maintenance unit and said humidifier, for connection to the inspirator line of a patient, a pressure control valve being arranged on an expirator line of a patient.

Further characteristics and advantages will become apparent from the description of a preferred but not exclusive embodiment of an apparatus for continuous alveolar positive pressure breathing, illustrated only by way of non-limitative example in the accompanying drawings, wherein:

figure 1 is a schematic view of the apparatus according to the invention;

figure 2 is a view of a different embodiment of the constant pressure maintenance unit with oscillating plates;

figure 3 is a view of different means for setting the generated pressure, which can be applied to the constant pressure maintenance unit of figure 2;

figure 4 is a view of a pressure maintenance unit with slidably movable plates.

With reference to the above figures, the apparatus for continuous alveolar positive pressure breathing, according to the invention, comprises a mixer, generally indicated by the reference numeral 1, which is constituted by at least one rotameter 2 for air, at least one rotameter 3 for oxygen and a possible auxiliary rotameter indicated by the reference numeral 4.

The mixer produces, on the line 5, a flow of air/oxygen mixture which is fed into a divider, constituted by a distribution valve 10, which creates in output a variable ratio between the flows: a first flow is fed, preferably by means of a first rotameter 11, into a constant pressure maintenance unit, generally indicated by the reference numeral 20, which will be described in greater detail hereinafter, while a second flow is fed into a humidifier 13, preferably by means of a second rotameter 12.

The distribution valve 10 has the purpose of setting the proportions of the ideal flows to be sent to the humidifier and to the constant pressure maintenance unit, so as to have in the humidifier a flow of gas which is always compatible with the characteristics of the humidifier itself.

The flow in output from the humidifier 13 and the flow in output from the constant pressure maintenance unit 20 merge, by means of a connection 21, on the inspirator line 22 which is connectable to a patient.

A pressure detector, generally indicated by the reference numeral 30, is furthermore provided on the inspirator line, whereas a pressure control valve 26, which is of the type commonly termed PEEP, is provided on the expirator line indicated by 25.

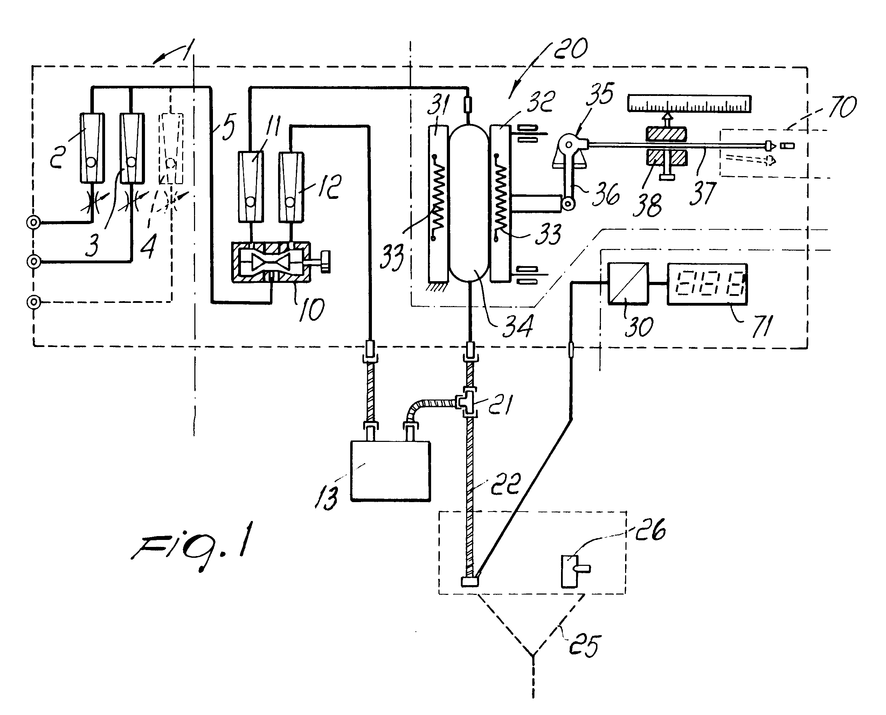

The constant pressure maintenance unit 20, as indicated in figure 1, is constituted by a first plate 31 and by a second plate 32 which are mutually opposite and have thermostat-controlled heating resistors 33 so as to create a constant physiological temperature of approximately 35-36° Centigrade; a bag 34 which acts as reserve and compensation tank is arranged between the plates.

One of the plates, for example the second plate 32, can slide so as to exert a pressure on the bag, so as to achieve the required degree of pressure.

As illustrated in figure 1, the pressure is obtained by means of an L-shaped lever 35 which is pivoted at its vertex and has one arm 36 which acts against the plate and another lever or arm 37 on which a weight 38 is placed; said weight is adjustable so as to exert the required pressure.

According to what is illustrated in figure 2, it is also possible to provide a different embodiment, constituted for example by a first and a second oscillating plate, indicated respectively by the reference numeral 41 and 42, which are articulated at one of their sides and retain, inside them, the bag, which is again indicated by the reference numeral 34.

The pressure is exerted by means of a variable weight 43 supported by a crosspiece 44 retained by cables 45 wound around pulleys 46, which are associated with the second plate and have a fixed point at 47 on the first plate.

In practice, the pressure exerted on the plate is varied by varying the weight 43.

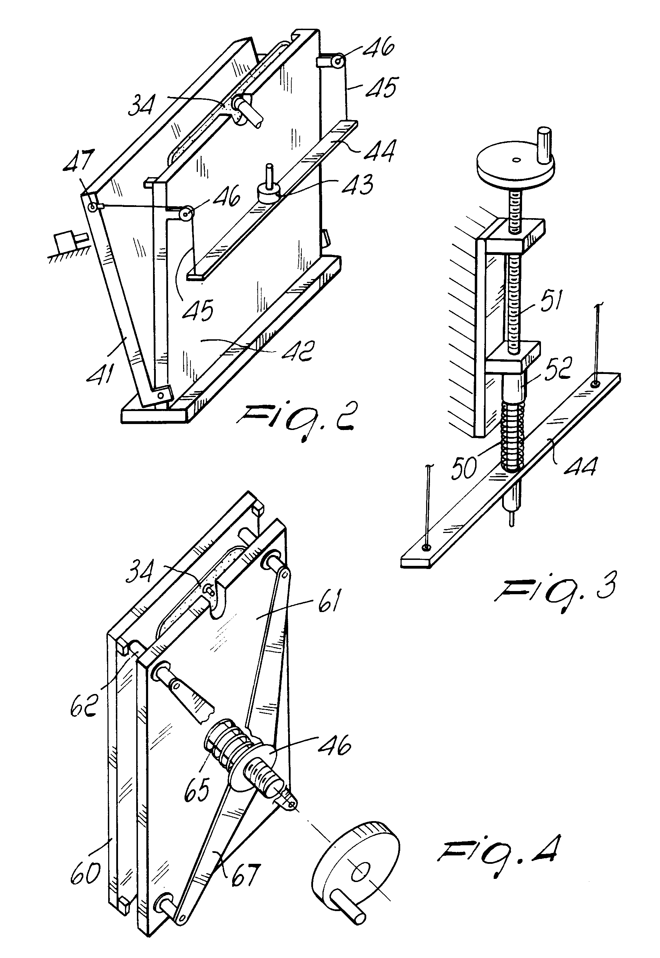

The weight adjustment system, as indicated in figure 3, can possibly be constituted by a pusher spring 50 which acts on the crosspiece 44, and can be set by means of a threaded bar 51 which causes the translatory motion of a female-thread slider 52 against which the pusher spring 50 abuts.

Figure 4 illustrates a different embodiment of the pressure maintenance unit, which has a fixed plate 60 and a slidably movable plate 61 which can slide on columns 62 which protrude from the fixed plate 60.

A compression spring 65 acts on the slidably movable plate 61 and is pushed by a bush 66, the height whereof can be adjusted so as to vary the pre-loading of the spring 65; the bush 66 is supported by spokes or braces 67 which are associated with the columns.

Another aspect of the invention is furthermore constituted by the fact that there is a patient apnea detection unit, which can be constituted by a mechanical detector 70, connected for example to the oscillating lever 37, which in practice detects the oscillation determined by the pressure drop for each inspiration act and therefore is capable of activating the alarm when respiration ceases.

It is similarly possible to provide an electronic detector 71 connected to the pressure detector 30 which is capable of activating the alarm when the pressure detector fails to detect the pressure variation determined by inspiration.

From what has been described above it can thus be seen that the invention achieves the intended aim and objects, and in particular the fact is stressed that the provision of the pressure maintenance unit by means of a bag which is retained between the two mutually movable plates allows to adjust with extreme precision and ease the pressure which is exerted, without having to resort to considerable volumes, as instead occurred with the elastic bags of the known art.

The throttling of the flows at the inlet of the humidifier furthermore allows to optimize the operation of said humidifier, since said humidifier receives a flow of gas which is compatible with the type and characteristics of the humidifier, thus allowing perfect operation.

The air is then heated by the plates of the pressure maintenance unit, with the consequent possibility of having a prolonged heat exchange and is consequently capable of adapting to all the operating characteristics.

Another important aspect is furthermore constituted by the fact that the apparatus allows to detect the apnea condition of the patient, since an absence of inspiration, which creates a variation in the operating pressure of said apparatus, is detected.

The invention thus conceived is susceptible to numerous modifications and variations, all of which are within the scope of the inventive concept.

All the details may furthermore be replaced with other technically equivalent elements.

In practice, the materials employed, as well as the dimensions and the contingent shapes, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

**Claims**

1. Apparatus for continuous alveolar positive pressure breathing, characterized in that it comprises, downstream of an air and oxygen mixer, a flow divider connected to a variable-volume constant pressure maintenance unit and to a humidifier, a merge connection being provided, downstream of said maintenance unit and of said humidifier, for connection to an inspirator line of a patient, a pressure control valve being arranged on the expirator line of a patient.

2. Apparatus according to the preceding claim, characterized in that said air or oxygen mixer comprises at least one rotameter for air, at least one rotameter for oxygen and an optional auxiliary rotameter.

3. Apparatus according to the preceding claims, characterized in that said flow divider is constituted by a distribution valve with a variable ratio of the output flows to set the proportions of the ideal flows to be sent to said humidifier and to said constant pressure maintenance unit.

4. Apparatus according to one or more of the preceding claims, characterized in that said constant pressure maintenance unit is constituted by a pair of opposite and mutually movable plates between which a bag, interposed on the inspirator line of the patient, is arranged.

5. Apparatus according to one or more of the preceding claims, characterized in that said plates have heating means.

6. Apparatus according to one or more of the preceding claims, characterized in that said constant pressure maintenance unit comprises a first and a second plate which face one another and between which a bag is interposed, an arm of an oscillating L-shaped lever acting on one of said plates, said lever being pivoted at its vertex and having, on its other arm, a weight which can be arranged so as to adjust the exerted pressure, elastic return means being furthermore provided.

7. Apparatus according to one or more of the preceding claims, characterized in that said constant pressure maintenance unit comprises a first and a second plate which oscillate and are mutually pivoted at one of their sides, a device for exerting pressure being furthermore provided and being constituted by a crosspiece on which a variable weight can be arranged, said crosspiece being supported, at its ends, by cables which wind around pulleys provided on one of said plates and have a fixed point on the opposite plate.

8. Apparatus according to one or more of the preceding claims, characterized in that said pressure unit has a pusher spring which acts on said crosspiece and abuts on a female thread connected to a threaded bar for the translatory motion of said female thread to vary the preloading of said pusher spring.

9. Apparatus according to one or more of the preceding claims, characterized in that said constant pressure maintenance unit comprises a fixed plate and a plate which can move translatorily on columns which extend from said fixed plate, a compression spring acting on said translatorily movable plate and abutting on a bush supported by spokes connected to said columns, a handwheel being provided to vary the position and the pre-loading of said compression spring.

10. Apparatus according to one or more of the preceding claims, characterized in that it comprises a patient apnea detection unit for monitoring the oscillating mechanical movement of an element which is associated with said constant pressure maintenance unit.

11. Apparatus according to one or more of the preceding claims, characterized in that it comprises a patient apnea detection device associated with the electronic manostat for controlling the pressure on the inspirator line.

Fig. 1

Fig. 2

Fig. 3

Fig. 4

# EUROPEAN SEARCH REPORT

EP 91 11 7664

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 204 535 (POHLMAN)<br>* abstract; figures *<br>* column 2, line 18 - line 28 * * <br>– – – | 1 | A 61 M 16/00 |
| A | US-A-4 621 633 (BOWLES ET AL.)<br>* abstract; claim 1; figure 10 * *<br>– – – | 1 | |
| A | DE-A-1 917 774 (VEB MEDIZINTECHNIK LEIPZIG)<br>* page 3, line 24 - page 5, line 12; figures * *<br>– – – | 1 | |
| A | EP-A-0 348 019 (THE BOC GROUP PLC)<br>* abstract; figures 1,2 *<br>* column 2, line 38 - column 3, line 26 * *<br>– – – | 1 | |
| A | US-A-4 575 042 (GRIMLAND ET AL.)<br>* abstract; figures * *<br>– – – | 1 | |
| A | W. W. MUSHIN 'Automatic Ventilation of the Lungs' 1980 , BLACKWELL SCIENTIFIC PUBLICATIONS , OXFORD LONDON EDINBURGH MELBOURNE<br>* page 770 - page 775 * *<br>– – – – – | 1 | |

**TECHNICAL FIELDS SEARCHED (Int. Cl.5)**

A 61 M

The present search report has been drawn up for all claims

| Place of search | Date of completion of search | Examiner |
|---|---|---|
| The Hague | 27 December 91 | ZEINSTRA H.S.J.H. |